# EUROPEAN PATENT APPLICATION

(11) **EP 4 324 549 A1**
(43) Date of publication of application: **21.02.2024**
(21) Application number: 22820248.7
(22) Date of filing: 08.06.2022
(51) Int. Cl.: B01D 69/04, B01D 53/22

(54) **METHOD FOR RECYCLING UNUSED GAS IN MEMBRANE REACTOR**

(30) Priority: 11.06.2021 JP 2021097904
(71) Applicant: JFE Steel Corporation, Tokyo 100-0011 (JP)
(72) Inventor: NISHIKAWA, Yuta, Tokyo 100-0011 (JP); SHIGAKI, Nobuyuki, Tokyo 100-0011 (JP)
(74) Representative: Grünecker Patent- und Rechtsanwälte PartG mbB
(86) International application number: PCT/JP2022/023097
(87) International publication number: WO 2022/260069

(57) **Abstract**

The purpose of the present invention is to provide a method for recycling an unused gas in a membrane reactor, the method making it possible to reduce production cost of a target product by recycling H2 gas permeating a separation membrane and flowing out from the membrane reactor. This method for recycling an unused gas in a membrane reactor includes: a gas phase reaction step in which a raw material gas containing hydrogen gas and at least one of carbon monoxide gas and carbon dioxide gas is introduced into a first space of a membrane reactor including a separation membrane, the first space being on a non-permeation side and filled with a catalyst, and a second space being on a permeation side, to synthesize a reaction product other and water vapor; a product membrane separation step in which at least one of the reaction product and the water vapor is allowed to permeate the separation membrane and enter the second space for separating; a mixed gas flowing out step in which a sweeping gas is flowed through the second space to cause hydrogen gas entering the second space to flow out; and a hydrogen gas recycling step in which at least hydrogen gas in the mixed gas flowed out is used as the raw material gas of the gas phase reaction step or a raw material gas of another process.

## Description

### TECHNICAL FIELD

The present invention relates to a method for recycling an unused gas in a membrane reactor.

### BACKGROUND ART

In recent years, the development of a technology called carbon capture and utilization (CCU) in which carbon dioxide (hereinafter, also referred to as "CO₂") gas is chemically reacted with hydrogen (hereinafter, also referred to as "H₂") gas to be converted into a valuable material for recycling has been studied as one of carbon dioxide emission reduction technologies.

The CCU technology includes, for example, a conversion technique to a chemical product or a fuel, and examples of a chemical reaction to be used include a reaction obtained by combining a reverse water gas shift reaction (formula (1)) and an existing method such as methanol synthesis (formula (2)) or Fischer-Tropsch synthesis (formula (3)) using carbon monoxide (hereinafter, also referred to as "CO") as a raw material.

CO₂+H₂⇔CO+H₂O (1)

CO+2H₂⇔CH₃OH (2)

nCO+ (m+n) H₂⇔CₙH₂ₘ+nH₂O (3)

In many CCU technologies, an equilibrium is involved with a chemical reaction, so that there is a thermodynamic upper limit. Therefore, in order to increase the yield, it is necessary to recycle an unreacted gas. Furthermore, these require a separation facility for water vapor that is simultaneously produced with a valuable material (hereinafter, also referred to as "target product") such as methanol or a hydrocarbon, which causes a problem in that the facility is increased in size.

One approach for breaking through the thermodynamic upper limit is a method in which a reaction equilibrium is shifted toward a product side by separating a part or all of a product from a reaction system. For example, in the methanol synthesis, a process has been proposed, in which a product is separated from a reaction system by a membrane reactor provided with a zeolite membrane, thereby breaking through the thermodynamic upper limit (Patent Literatures 1 to 3) .

### CITATION LIST

### PATENT LITERATURE

Patent Literature 1: JP 2016-174996 A
Patent Literature 2: JP 2018-008940 A
Patent Literature 3: JP 2020-023488 A
Patent Literature 4: JP 2020-132439 A

### SUMMARY OF INVENTION

### TECHNICAL PROBLEMS

H₂ gas used in the above process can be produced, for example, by electrolysis of water using electric power derived from renewable energy. There are various kinds of renewable energy, for example, solar power, wind power, and hydraulic power. Since the price of H₂ gas produced from the renewable energy depends on the power price of the renewable energy, the implementation of the CCU technology in which the H₂ gas price greatly affects the production cost is economically difficult in a place where the renewable energy is not inexpensively available. However, the economic efficiency of H₂ gas is not examined in any of the above literatures.

In order to obtain H₂ gas at low cost, for example, a method in which H₂ is produced by effectively using water vapor separated from reaction heat in a membrane reactor has been proposed (Patent Literature 4). In the Literature, in order to recover gas and heat separated by the membrane reactor, a method is proposed, in which carbon monoxide gas or hydrocarbon-based gas is used as a sweeping gas, and heat is then recovered by a water gas shift reaction or a steam reforming reaction which is an endothermic reaction, while H₂ gas is produced.

Under such circumstances, the study of the present inventors revealed that a separation membrane included in a membrane reactor selectively allows a specific compound to permeate such that the compound is separated from the reaction system, but the permeation of gas other than the specific compound is also not zero, and in addition to at least one of a target product and water vapor as the specific compound, H₂ gas, carbon monoxide gas, and carbon dioxide gas which are raw material gases partially permeate from the reaction system together with the product. In particular, it has been revealed that it is difficult to suppress the permeation of H₂ gas because of its small molecular diameter. H₂ gas separated from the reaction system through the separation membrane is not involved with a gas phase reaction and becomes an unused gas.

In addition, the chemical reaction including the combination of the formula (1) and the formula (2) or the combination of the formula (1) and the formula (3) described as the CCU technology is performed under a high pressure condition due to the above-described thermodynamic restriction. Considering a stoichiometric ratio, the substance amount of H₂ gas accounts for at least half or more of the raw material gas. Since a driving force for permeation through the separation membrane is a partial pressure difference between a permeation side and a non-permeation side, the condition that a large amount of H₂ gas is contained under such high pressure is a very disadvantageous condition in suppressing the permeation of H₂ gas.

For the above two reasons, there is a problem in that the utilization efficiency of H₂ gas that greatly affects the production cost of the CCU technology decreases, and the economic efficiency deteriorates.

The present invention has been made in view of the above problems, and an object of the present invention is to provide a method for recycling an unused gas in a membrane reactor, which method makes it possible to reduce the production cost of a target product in the membrane reactor by recycling unused H₂ gas permeating a separation membrane and flowing out from the membrane reactor.

### SOLUTION TO PROBLEMS

As described above, the study of the present inventors revealed that a part of hydrogen gas as the raw material gas permeates the separation membrane of the membrane reactor. The present invention is based on this finding, and the following configuration specifically solves the above problems.

(1) A method for recycling an unused gas in a membrane reactor, the method comprising:
   a gas phase reaction step in which a raw material gas containing hydrogen gas and at least one of carbon monoxide gas and carbon dioxide gas is introduced into a first space (E1) of a membrane reactor (E) including a separation membrane (M), the first space (E1) being on a non-permeation side and filled with a catalyst, and a second space (E2) being on a permeation side, to synthesize a reaction product other than water vapor and water vapor by an action of the catalyst;
   a product membrane separation step in which at least one product of the reaction product other than water vapor and the water vapor generated in the first space (E1) by the gas phase reaction step is allowed to permeate the separation membrane (M) and enter the second space (E2) for separating;
   a mixed gas flowing out step in which a sweeping gas is flowed through the second space (E2) to cause hydrogen gas as the raw material gas permeating the separation membrane (M) and entering the second space (E2) to flow out from the second space (E2) together with the product separated in the product membrane separation step; and
   a hydrogen gas recycling step in which at least hydrogen gas in a mixed gas flowed out from the second space (E2) in the mixed gas flowing out step is used as the raw material gas of the gas phase reaction step or a raw material gas of another process.
(2) The method for recycling an unused gas in a membrane reactor according to (1) above, further comprising, before the hydrogen gas recycling step:
   a mixed gas separation step in which the mixed gas flowed out in the mixed gas flowing out step is separated to increase a concentration of hydrogen gas.
(3) The method for recycling an unused gas in a membrane reactor according to (1) or (2) above, wherein a part or all of the mixed gas containing at least hydrogen gas flowed out in the mixed gas flowing out step is mixed with at least one gas selected from the group consisting of carbon monoxide gas, carbon dioxide gas, and hydrogen gas, and a composition and a flow rate of the mixture are adjusted to obtain the raw material gas for the gas phase reaction step.
(4) The method for recycling an unused gas in a membrane reactor according to any one of (1) to (3) above, wherein a part of the mixed gas flowed out in the mixed gas flowing out step is mixed with the sweeping gas and circularly used.
(5) The method for recycling an unused gas in a membrane reactor according to any one of (1) to (4) above, wherein the sweeping gas contains at least one gas selected from the group consisting of carbon monoxide gas, carbon dioxide gas, and hydrogen gas.
(6) The method for recycling an unused gas in a membrane reactor according to any one of (1) to (5) above, wherein the reaction product other than water vapor is methanol.

### ADVANTAGEOUS EFFECTS OF INVENTION

According to the present invention, it is possible to increase the utilization efficiency of H₂ gas as a raw material gas with respect to a membrane reactor provided with a separation membrane having a wide range of performance, thus effectively utilizing H₂ gas.

According to the present invention, unused H₂ gas permeating the separation membrane can be recycled, and therefore a product can be obtained at a lower cost than that in a conventional membrane reactor process.

### BRIEF DESCRIPTION OF DRAWINGS

[FIG. 1] FIG. 1 is a view showing one embodiment of a method for recycling an unused gas in a membrane reactor according to the present invention.
[FIG. 2] FIG. 2 is a view showing one example of a method for recycling an unused gas in a membrane reactor according to the present invention.

### DESCRIPTION OF EMBODIMENTS

Hereinafter, a method for recycling an unused gas in a membrane reactor according to the present invention will be described.

The fact that hydrogen gas can be effectively used and the fact that the yield of a target product is high are also referred to as "the effect and the like of the present invention are excellent".

A method for recycling an unused gas in a membrane reactor according to the present invention (hereinafter, also referred to as "method of the present invention") includes:
a gas phase reaction step in which a raw material gas containing hydrogen gas and at least one of carbon monoxide gas and carbon dioxide gas is introduced into a first space (E1) of a membrane reactor (E) including a separation membrane (M), the first space (E1) being on a non-permeation side and filled with a catalyst, and a second space (E2) being on a permeation side, to synthesize a reaction product other than water vapor and water vapor by an action of the catalyst;
a product membrane separation step in which at least one product of the reaction product other than water vapor and the water vapor generated in the first space (E1) by the gas phase reaction step is allowed to permeate the separation membrane (M) and enter the second space (E2) for separating;
a mixed gas flowing out step in which a sweeping gas is flowed through the second space (E2) to cause hydrogen gas as the raw material gas permeating the separation membrane (M) and entering the second space (E2), to flow out from the second space (E2) together with the product separated in the product membrane separation step; and
a hydrogen gas recycling step in which at least hydrogen gas in the mixed gas flowed out in the mixed gas flowing out step is used as the raw material gas of the gas phase reaction step or a raw material gas of another process.

First, the method of the present invention will be described with reference to the drawings.

### [Description with reference to drawings]

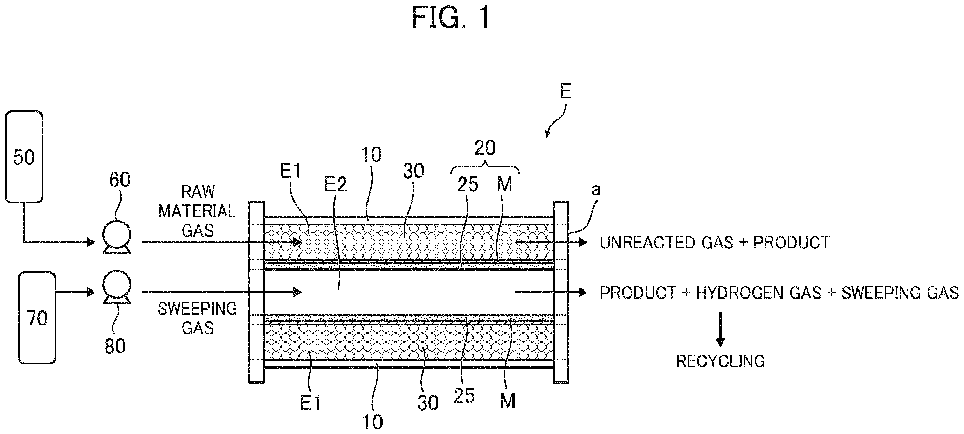

FIG. 1 is a view showing one embodiment of a method for recycling an unused gas in a membrane reactor according to the present invention.

In FIG. 1, a membrane reactor E has a double tube structure with a reaction tube 10 and a separation tube 20. A first space E1 (first space E1 on a non-permeation side) is defined between the reaction tube 10 and the separation tube 20, and a second space E2 (second space E2 on a permeation side) is defined inside the separation tube 20. Here, the separation tube 20 is composed of an inner tube 25 and a separation membrane M present on the surface, on the reaction tube 10 side, of the inner tube 25. The first space E1 is filled with a catalyst 30.

First, in the gas phase reaction step, a raw material gas containing hydrogen gas and at least one of carbon monoxide gas and carbon dioxide gas is introduced into the first space E1 of the membrane reactor E, whereby a reaction product other than water vapor (target product) and water vapor are synthesized by the action of the catalyst 30.

The reaction product and an unreacted gas (raw material gas that has not reacted) that have not permeated the separation membrane are flowed out from the outlet side a of the first space E1, and the target product is recovered.

In the product membrane separation step, at least one product of the target product and the water vapor generated in the first space E1 in the gas phase reaction step described above permeates the separation membrane M (separation tube 20) and enters the second space E2, and is thus separated.

In the mixed gas flowing out step, a sweeping gas is flowed through the second space E2 to cause hydrogen gas as the raw material gas permeating the separation membrane M (separation tube 20) and entering the second space E2, to flow out from the second space E2 together with the product separated in the product membrane separation step. The mixed gas flowed out from the second space E2 includes the product separated in the product membrane separation step, hydrogen gas as the raw material permeating the separation membrane M and entering the second space E2, and the sweeping gas.

In the hydrogen gas recycling step, at least hydrogen gas in the mixed gas flowed out in the mixed gas flowing out step is used as the raw material gas in the gas phase reaction step or a raw material gas in another process.

Next, the membrane reactor (E) used in the method of the present invention (hereinafter, also simply referred to as "membrane separator") will now be described in detail.

### [Membrane reactor]

The membrane reactor includes a separation membrane (M) (hereinafter, also simply referred to as "separation membrane"), a first space (E1) being on a non-permeation side (hereinafter, also simply referred to as "first space") and filled with a catalyst, and a second space (E2) being on a permeation side (hereinafter, also simply referred to as "second space").

### [Separation membrane]

The separation membrane is a membrane that allows at least one of the reaction product other than water vapor and the water vapor generated in the gas phase reaction step to permeate for separating.

The separation membrane is preferably a membrane that allows water vapor to permeate for separating for the reason that the effect and the like of the present invention are more excellent.

The separation membrane is preferably an inorganic membrane for the reason that the inorganic membrane has durability capable of maintaining chemical properties even under high-temperature and high-pressure conditions in which the reaction proceeds, and more preferably a zeolite membrane.

Examples of the zeolite membrane include an LTA type (A type zeolite) and an SOD type, and among them, the LTA type is preferable for the reason that the effect and the like of the present invention are more excellent.

Since hydrogen gas has a small molecular diameter, it usually undesirably permeates the separation membrane.

### [Separation tube]

The membrane separator preferably includes a separation tube composed of a porous (for example, porous alumina) inner tube and a separation membrane present on the surface of the inner tube for the reason that the effect and the like of the present invention are more excellent.

The separation membrane itself may function as the separation tube.

### [First space]

The first space is a space being on the non-permeation side of the separation membrane.

Usually, a membrane separation tube has a double structure with a reaction tube (outer tube) and a separation tube. In this case, the first space is a space between the reaction tube and the separation tube.

### <Catalyst>

As described above, the first space is filled with a catalyst.

The catalyst is not particularly limited as long as it is a catalyst for a gas phase reaction to be described later.

The catalyst is preferably a metal catalyst for the reason that the effect and the like of the present invention are more excellent.

In the case of the reaction of the formula (2) described above, the catalyst is preferably a copper-zinc-based catalyst for the reason that the effect and the like of the present invention are more excellent.

In the case of the reaction of the formula (3) described above, the catalyst is preferably an iron-based catalyst or a cobalt-based catalyst for the reason that the effect and the like of the present invention are more excellent.

### [Second space]

The second space is a space being on the permeation side of the separation membrane.

As described above, usually, the membrane separation tube has a double structure with a reaction tube (outer tube) and a separation tube. In this case, the second space is a space inside the separation tube.

Next, each step of the method of the present invention will be described in detail.

### [Gas phase reaction step]

The gas phase reaction step is a step in which a raw material gas containing hydrogen gas and at least one of carbon monoxide gas and carbon dioxide gas is introduced into a first space (E1) of a membrane reactor (E) including a separation membrane (M), the first space (E1) being on a non-permeation side and filled with a catalyst, and a second space (E2) being on a permeation side, to synthesize a reaction product other than water vapor (target product) and water vapor by an action of the catalyst.

Examples of the target product include methanol and paraffins and olefins which are products of Fischer-Tropsch synthesis, as mentioned with the formula (2) and the formula (3). When carbon dioxide is used as the raw material gas, a similar target product is also obtained through the formula (1).

The reaction product in the gas phase reaction step is not particularly limited, but the reaction product is particularly preferably methanol. The target product in the gas phase reaction step is particularly preferably methanol because of the following reason: when the reaction product is methanol, hydrogen and at least one of methanol and water vapor are recovered by the sweeping gas, and in a mixed gas separation step to be described later in which hydrogen gas is separated from at least one of methanol and water vapor, the concentration of the hydrogen gas can be increased by a simple and inexpensive gas-liquid separation method.

The obtained target product is flowed out from the first space and recovered.

### [Product membrane separation step]

The product membrane separation step is a step in which at least one product of the reaction product other than water vapor (target product) and water vapor generated in the first space (E1) in the gas phase reaction step is allowed to permeate the separation membrane (M) and enter the second space (E2) for separating.

In the product membrane separation step, at least one of the target product and water vapor synthesized in the gas phase reaction step is selectively separated. By removing the product synthesized in the gas phase reaction step from the reaction system and shifting the chemical reaction equilibrium toward the product side, the gas phase reaction efficiently proceeds.

When the product is generated in the first space by the gas phase reaction, the partial pressure of the product in the first space increases, and therefore the product permeates the separation membrane and enters the second space, and is thus separated. As described later, by flowing the sweeping gas through the second space, the product having entered the second space and thus separated is flowed out from the second space together with the sweeping gas, so that a difference (partial pressure difference) between the partial pressure of the product in the first space and the partial pressure of the product in the second space is maintained. That is, the permeation and entrance of the product into the second space is promoted by the flow of the sweeping gas.

### [Mixed gas flowing out step]

The mixed gas flowing out step is a step in which by flowing the sweeping gas through the second space (E2), hydrogen gas as the raw material gas permeating the separation membrane M and entering the second space (E2), is caused to flow out from the second space (E2) together with the product separated in the product membrane separation step. When the mixed gas flowed out from the second space contains the target product, this may be recovered.

### [Sweeping gas]

The sweeping gas is not particularly specified, but at least one of carbon monoxide gas, carbon dioxide gas, and hydrogen gas is preferable because the sweeping gas and the hydrogen gas permeating the separation membrane and entering the second space can be introduced as the raw material gas of the above-described gas phase reaction step without being separated.

When hydrogen gas is used as the sweeping gas, the sweeping gas and the hydrogen gas permeating the separation membrane and entering the second space can be used in the hydrogen gas recycling step without being separated, and therefore the sweeping gas is particularly preferably hydrogen gas.

For the sweeping gas, a part or all of the gas containing hydrogen gas flowed out from the second space (E2) is circulated as the sweeping gas and recycled, whereby the concentration of hydrogen gas recovered by the sweeping gas increases, and the total amount of gas used for the sweeping gas can be saved. Therefore, it is preferable that a part or all of the gas containing hydrogen gas flowed out from the second space (E2) be circulated as the sweeping gas and used.

### [Hydrogen gas recycling step]

The hydrogen gas recycling step is a step in which at least hydrogen gas in the mixed gas flowed out from the second space (E2) in the mixed gas flowing out step is used as the raw material gas in the gas phase reaction step or a raw material gas in another process.

Specific examples of another process include a power generation facility, and hydrogen gas can be used by being mixed with various raw material gases such as natural gas and blast furnace gas.

### [Mixed gas separation step]

The method of the present invention preferably further includes, before the hydrogen gas recycling step, a step (mixed gas separation step) in which the mixed gas flowed out in the mixed gas flowing out step is separated to increase the concentration of hydrogen gas for the reason that the effect and the like of the present invention are more excellent.

The separation method in the mixed gas separation step is not particularly specified, and any of a gas-liquid separation method using a difference between boiling points, an adsorption method using an adsorbent, a membrane separation method, and the like can be used. The separation method can be selected according to the composition of the mixed gas containing hydrogen gas recovered by the sweeping gas and a suitable gas composition in the hydrogen gas recycling step.

Before the mixed gas is introduced as the raw material gas of the gas phase reaction step in the hydrogen gas recycling step, it is preferable to adjust the composition and flow rate of hydrogen gas and at least one of the carbon monoxide gas and the carbon dioxide gas used for the raw material gas in the gas phase reaction step by mixing at least one of carbon monoxide gas, carbon dioxide gas, and hydrogen gas with the mixed gas because if the composition and flow rate of the hydrogen gas and at least one of the carbon monoxide gas and the carbon dioxide gas of the raw material gas are adjusted to values suitable for the gas phase reaction, the utilization efficiency of hydrogen gas in the gas phase reaction can be improved.

### [Suitable embodiment]

In the method of the present invention, for the reason that the effect and the like of the present invention are more excellent, it is preferable that the separation membrane in the membrane reactor be a separation membrane that allows water vapor to permeate (water vapor separation membrane) (preferably, a membrane that hardly allows the target product to permeate); the sweeping gas used in the mixed gas flowing out step be hydrogen gas; the method further include the mixed gas separation step (a step in which the mixed gas (water vapor, hydrogen gas) flowed out in the mixed gas flowing out step is separated by a gas-liquid separation method to increase the concentration of hydrogen gas) before the hydrogen gas recycling step; a part of the gas (gas obtained by concentrating the hydrogen gas) obtained in the mixed gas separation step be mixed with carbon monoxide or carbon dioxide to obtain the raw material gas for the gas phase reaction step; and the remaining gas obtained in the mixed gas separation step be used as the sweeping gas. According to such an embodiment, hydrogen gas permeating the separation membrane can be recycled, and therefore the yield of the target product (for example, methanol) is further improved.

### EXAMPLES

Hereinafter, the present invention will be described in more detail with reference to Examples, but the present invention is not limited thereto.

### [Example 1]

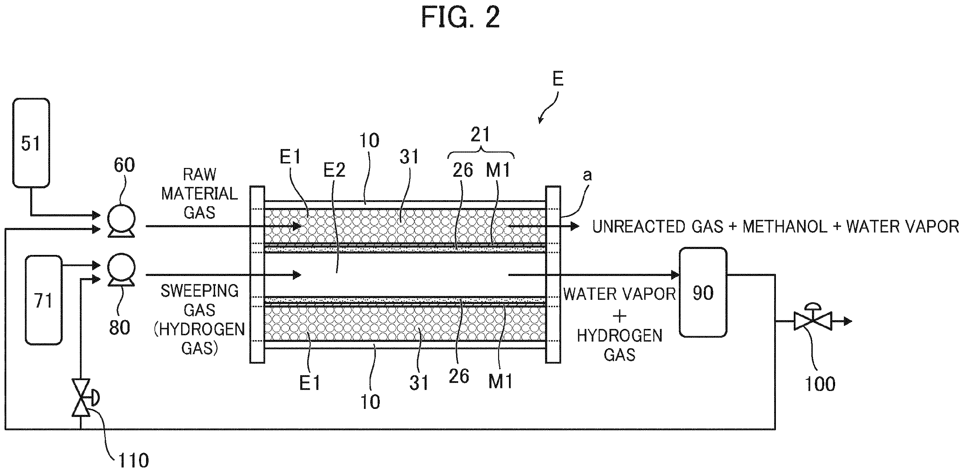

FIG. 2 is a view showing one example of the method of the present invention.

In FIG. 2, a membrane reactor E has a double tube structure with a reaction tube 10 and a separation tube 21. A first space E1 (first space E1 being on a non-permeation side) is defined between the reaction tube 10 and the separation tube 21, and a second space E2 (second space E2 being on a permeation side) is defined inside the separation tube 21. The separation tube 21 is composed of a porous alumina tube (inner tube) 26 and a water vapor separation membrane M1 (obtained by precipitating LTA type zeolite) present on the surface, on the reaction tube 10 side, of the alumina tube 26. The first space E1 is filled with a copper-zinc-based catalyst 31.

A raw material gas (carbon dioxide gas, hydrogen gas) was introduced into the first space E1 by a compressor 60 at 5 MPaA and 200°C, and a sweeping gas (hydrogen gas) was introduced into the second space E2 inside the separation tube 21 by a blower 80 at normal pressure and 160°C.

### <Gas phase reaction step>

The raw material gas containing carbon dioxide gas and hydrogen gas was introduced into the first space E1. By introducing the raw material gas into the first space E1, methanol (reaction product other than water vapor) (target product) and water vapor were synthesized by the action of the catalyst 31.

An unreacted gas (raw material gas that did not react) and the product (methanol, water vapor) that remained in the first space E1 without permeating the separation membrane were flowed out from the outlet side a of the first space E1, and methanol was recovered.

### <Product membrane separation step>

Of the product generated in the first space in the gas phase reaction step, the water vapor permeated the water vapor separation membrane M1 and entered the second space E2 for separating.

### <Mixed gas flowing out step>

A sweeping gas (hydrogen gas) was flowed through the second space E2. Consequently, hydrogen gas as the raw material gas having permeated the water vapor separation membrane M1 and entered the second space E2 together with the water vapor having been separated in the product membrane separation step was flowed out from the second space E2. Hydrogen gas of 1 NL/min (minute) was used as the sweeping gas. The mixed gas flowed out from the second space E2 in the mixed gas flowing out step included the water vapor having been separated in the product membrane separation step, the hydrogen gas as the raw material gas having permeated the water vapor separation membrane M1 and entered the second space E2, and the hydrogen gas as the sweeping gas.

### <Mixed gas separation step>

The water vapor of the mixed gas flowed out in the mixed gas flowing out step was separated by a gas-liquid separator 90, to concentrate hydrogen gas.

### <Hydrogen gas recycling step>

0.25 NL/min of the obtained gas (gas obtained by concentrating the hydrogen gas) was used as a part of 1 NL/min of a sweeping gas (hydrogen gas) introduced into the second space E2 of the membrane reactor E via a flow rate regulation valve 110. The remaining gas obtained by subtracting 0.25 NL/min from the obtained gas was mixed with 0.25 NL/min of carbon dioxide gas. Then, the pressure of the mixed gas was increased by the compressor 60, and the mixed gas was used as the raw material gas to be introduced into the first space E1 of the membrane reactor E (the raw material gas of the gas phase reaction step described above).

### [Comparative Example 1]

The same membrane reactor E as in Example 1 described above was used.

A raw material gas (0.25 NL/min of carbon dioxide gas and 0.75 NL/min of hydrogen gas) was introduced into the first space E1 by the compressor 60 at 5 MPaA and 200°C, and a sweeping gas (1 NL/min of nitrogen gas) was introduced into the second space E2 inside the separation tube 21 by the blower 80 at normal pressure and 160°C.

A gas phase reaction step and a product membrane separation step are the same as those in Example 1 described above.

The sweeping gas (nitrogen gas) was flowed through the second space E2. Consequently, hydrogen gas as the raw material gas having permeated the water vapor separation membrane M1 and entered the second space E2 together with the water vapor having been separated in the product membrane separation step was flowed out from the second space E2. The mixed gas flowed out from the second space E2 included the water vapor having been separated in the product membrane separation step, the hydrogen gas as the raw material gas having permeated the water vapor separation membrane M1 and entered the second space E2, and the hydrogen gas as the sweeping gas.

The mixed gas flowed out in the mixed gas flowing out step was wholly discharged from the flow rate regulation valve 100, without being recycled.

The raw material gas (carbon dioxide gas, hydrogen gas) used in the entire process is the same in Example 1 and Comparative Example 1.

### [Hydrogen gas flow rate]

Table 1 below shows hydrogen gas flow rates at the inlet and outlet of the first space E1 and second space E2 of the membrane reactor E in Example 1 and Comparative Example 1.

### [Methanol yield]

The methanol yields of Example 1 and Comparative Example 1 are shown in the following Table 1.

Here, the methanol yield represents the ratio of the amount of produced methanol to the amount of carbon dioxide gas introduced as the raw material gas.

**[Table 1]**

| Table 1 | Hydrogen gas flow rate | | | | Methanol yield [%] |
|---|---|---|---|---|---|
| | E1 Inlet [NL/min] | E1 Outlet [NL/min] | E2 Inlet [NL/min] | E2 Outlet [NL/min] | |
| Comparative Example 1 | 0.75 | 0.11 (Decrease due to reaction and permeation) | 0.00 | 0.14 (Increase due to permeation) | 60 |
| Example 1 | 0.89 | 0.15 (Decrease due to reaction and permeation) | 1.00 | 1.14 (Increase due to permeation) | 73 |

As can be seen from Table 1, the methanol yield of Example 1 was 73%, and the methanol yield of Comparative Example 1 was 60%. As compared with Comparative Example 1, it was found that in Example 1, the methanol yield was high, and hydrogen gas having entered the second space E2 was effectively used. In Comparative Example 1, hydrogen gas remained as an unused gas in the mixed gas flowed out from the second space E2.

### DESCRIPTION OF SYMBOLS

- 10: Reaction tube
- 20: Separation tube
- 25: Inner tube
- 26: Porous alumina tube (inner tube)
- 30: Catalyst
- 31: Copper-Zinc-based catalyst
- 50: Raw material gas tank
- 51: Carbon dioxide gas tank
- 60: Compressor
- 70: Sweeping gas tank
- 71: Hydrogen gas tank
- 80: Blower
- 90: Gas-liquid separator
- 100: Flow rate control valve
- 110: Flow rate control valve
- E: Membrane reactor
- E1: First space (catalyst packed bed)
- a: Outlet side of first space E1
- E2: Second space
- M: Separation membrane
- M1: Water vapor separation membrane

## Claims

1. A method for recycling an unused gas in a membrane reactor, the method comprising:
a gas phase reaction step in which a raw material gas containing hydrogen gas and at least one of carbon monoxide gas and carbon dioxide gas is introduced into a first space (E1) of a membrane reactor (E) including a separation membrane (M), the first space (E1) being on a non-permeation side and filled with a catalyst, and a second space (E2) being on a permeation side, to synthesize a reaction product other than water vapor and water vapor by an action of the catalyst;
a product membrane separation step in which at least one product of the reaction product other than water vapor and the water vapor generated in the first space (E1) by the gas phase reaction step is allowed to permeate the separation membrane (M) and enter the second space (E2) for separating;
a mixed gas flowing out step in which a sweeping gas is flowed through the second space (E2) to cause hydrogen gas as the raw material gas permeating the separation membrane (M) and entering the second space (E2) to flow out from the second space (E2) together with the product separated in the product membrane separation step; and
a hydrogen gas recycling step in which at least hydrogen gas in a mixed gas flowed out from the second space (E2) in the mixed gas flowing out step is used as the raw material gas of the gas phase reaction step or a raw material gas of another process.

2. The method for recycling an unused gas in a membrane reactor according to claim 1, further comprising, before the hydrogen gas recycling step:
a mixed gas separation step in which the mixed gas flowed out in the mixed gas flowing out step is separated to increase a concentration of hydrogen gas.

3. The method for recycling an unused gas in a membrane reactor according to claim 1 or 2, wherein a part or all of the mixed gas containing at least hydrogen gas flowed out in the mixed gas flowing out step is mixed with at least one gas selected from the group consisting of carbon monoxide gas, carbon dioxide gas, and hydrogen gas, and a composition and a flow rate of the mixture are adjusted to obtain the raw material gas for the gas phase reaction step.

4. The method for recycling an unused gas in a membrane reactor according to any one of claims 1 to 3, wherein a part of the mixed gas flowed out in the mixed gas flowing out step is mixed with the sweeping gas and circularly used.

5. The method for recycling an unused gas in a membrane reactor according to any one of claims 1 to 4, wherein the sweeping gas contains at least one gas selected from the group consisting of carbon monoxide gas, carbon dioxide gas, and hydrogen gas.

6. The method for recycling an unused gas in a membrane reactor according to any one of claims 1 to 5, wherein the reaction product other than water vapor is methanol.
